# EUROPEAN PATENT SPECIFICATION

(11) **EP 3 678 254 B1**
(45) Date of publication and mention of the grant of the patent: **28.02.2024**
(21) Application number: 19150356.4
(22) Date of filing: 04.01.2019
(51) Int. Cl.: H01M 10/42, H01M 10/48, H01M 50/317, H01M 10/052

(54) **METHOD FOR DETERMINING THE COMPOSITION OF A GASEOUS MIXTURE ENCLOSED WITHIN A GAS-TIGHT HOUSING OF A BATTERY PACK AND A CORRESPONDING GAS-TIGHT HOUSING**
VERFAHREN ZUR BESTIMMUNG DER ZUSAMMENSETZUNG EINER GASMISCHUNG, DIE IN EINEM GASDICHTEN GEHÄUSE EINES BATTERIEPACKS EINGESCHLOSSEN IST, UND ZUGEHÖRIGES GASDICHTES GEHÄUSE
PROCÉDÉ DE DÉTERMINATION DE LA COMPOSITION D'UN MÉLANGE GAZEUX CONTENU DANS UN LOGEMENT ÉTANCHE AUX GAZ D'UN BLOC-BATTERIE ET LOGEMENT ÉTANCHE AUX GAZ CORRESPONDANT

(43) Date of publication of application: 08.07.2020
(73) Proprietor: Samsung SDI Co., Ltd., Gyeonggi-do 17084 (KR)
(72) Inventor: Erhart, Michael, 8054 Seiersberg-Pirka (AT)
(74) Representative: Gulde & Partner

(56) References cited:
- DE-A1-102014 222 786
- US-A1- 2014 174 150

## Description

### Field of the Invention

The present invention relates to a method for determining the composition of a gaseous mixture enclosed within a gas-tight housing of a battery pack.

### Technological Background

In the recent years, vehicles for transportation of goods and peoples have been developed using electric power as a source for motion. Such an electric vehicle is an automobile that is propelled by an electric motor, using energy stored in rechargeable batteries. An electric vehicle may be solely powered by batteries or may be a form of hybrid vehicle powered by for example a gasoline generator. Furthermore, the vehicle may include a combination of electric motor and conventional combustion engine. In general, an electric-vehicle battery (EVB) or traction battery is a battery used to power the propulsion of battery electric vehicles (BEVs). Electric-vehicle batteries differ from starting, lighting, and ignition batteries because they are designed to give power over sustained periods of time. A rechargeable or secondary battery differs from a primary battery in that it can be repeatedly charged and discharged, while the latter provides only an irreversible conversion of chemical to electrical energy. Low-capacity rechargeable batteries are used as power supply for small electronic devices, such as cellular phones, notebook computers and camcorders, while high-capacity rechargeable batteries are used as the power supply for hybrid vehicles and the like.

In general, rechargeable batteries include an electrode assembly including a positive electrode, a negative electrode, and a separator interposed between the positive and negative electrodes, a case receiving the electrode assembly, and an electrode terminal electrically connected to the electrode assembly. An electrolyte solution is injected into the case in order to enable charging and discharging of the battery via an electrochemical reaction of the positive electrode, the negative electrode, and the electrolyte solution. The shape of the case, e.g. cylindrical or rectangular, depends on the battery's intended purpose. Lithium-ion (and similar lithium polymer) batteries, widely known via their use in laptops and consumer electronics, dominate the most recent group of electric vehicles in development.

Rechargeable batteries may be used as a battery module formed of a plurality of unit battery cells coupled in series and/or in parallel so as to provide a high energy density, in particular for motor driving of a hybrid vehicle. That is, the battery module is formed by interconnecting the electrode terminals of the plurality of unit battery cells depending on a required amount of power and in order to realize a high-power rechargeable battery.

A battery pack is a set of any number of (preferably identical) battery modules. They may be configured in a series, parallel or a mixture of both to deliver the desired voltage, capacity, or power density. Components of battery packs include the individual battery modules, and the interconnects, which provide electrical conductivity between them.

The mechanical integration of such a battery pack requires appropriate mechanical connections between the individual components, e.g. of battery modules, and between them and a supporting structure of the vehicle. These connections must remain functional and save during the average service life of the battery system. Further, installation space and interchangeability requirements must be met, especially in mobile applications.

Mechanical integration of battery modules may be achieved by providing a carrier framework and by positioning the battery modules thereon. Fixing the battery cells or battery modules may be achieved by fitted depressions in the framework or by mechanical interconnectors such as bolts or screws. Alternatively, the battery modules are confined by fastening side plates to lateral sides of the carrier framework. Further, cover plates may be fixed atop and below the battery modules.

The carrier framework of the battery pack is mounted to a carrying structure of the vehicle. In case the battery pack shall be fixed at the bottom of the vehicle, the mechanical connection may be established from the bottom side by for example bolts passing through the carrier framework of the battery pack. The framework as well as the cover plates fixed atop and below the battery modules are usually made of aluminum or an aluminum alloy to lower the total weight of the construction.

Battery packs according to the prior art, despite any modular structure, usually comprise a battery pack housing that serves as enclosure to seal the battery pack against the environment and provides structural protection of the battery pack's components. Housed battery packs are usually mounted as a whole into their application environment, e.g. an electric vehicle. Li-ion battery modules for EV's are placed inside the common battery pack housing.

Battery packs for vehicles are designed to withstand crash events. However crash events or environmental conditions, for example flooding, could be very severe and may lead to destruction of internal parts of the battery pack, for example rupture of battery cells, electric shorts or fire. External diagnostic of the health state of the battery pack may not be possible anymore through electronic analyses (i.e. read out of fault memory) and certain faults (for example opening of single cells and rupture of cell contents) can not be reliable detected by battery electronics. Thus, the safety status of the battery may be unknown.

US 2014 / 174150 A1 discloses battery modules used as a battery pack for an electric vehicle. It further discloses a method comprising disposing a gas sensor with the gas sensor facing a first opening that is provided in the case; compulsorily introducing air into the case through a second opening that is provided in another position of the case; and judging the presence or absence of the leak of the film-sheathed battery from an output of the gas sensor.

It is an object of the present invention to overcome or reduce at least some of the drawbacks of the prior art and to provide a testing method that allows determination of the safety status of the battery pack even under extraordinary conditions.

### Summary of Invention

Embodiments of the present disclosure seek to solve at least one of the problems existing in the prior art to at least some extent. In particular, a method for determining the composition of a gaseous mixture enclosed within a gas-tight housing of a battery pack for a vehicle is provided, wherein the housing includes an air inlet port and an air outlet port, which are sealed by removable sealing means. The sealing means are screwed sealing plugs. The method comprises the steps of:
a) removing the screwed sealing plugs from the air inlet port and from the air outlet port;
b) generating an air stream entering the housing through the air inlet port and leaving the housing at the air outlet port; and
c) analyzing the air stream leaving the air outlet port with a gas analyzer.

A main aspect of the present invention is to provide a testing method, which helps to analyze the state of health of a vehicle's battery pack, in special operation situations. For example, due to a crash, measurement electronics inside the battery pack may not work anymore and the battery pack status is unclear. Special operation situations, where the test system may be useful, include for example:
- checking a battery pack status directly after a crash by a rescue team;
- determination of information whether or not the battery pack can be transported after a crash;
- performing an initial check in repair stations after a crash or in unclear situations;
- checking H₂ gas release in case of leakage of the battery cooling system within the battery pack housing;
- precautionary measure in case of regular inspection of the battery pack; and
- output inspection of battery packs prior to delivery to the customer.

The battery pack thus involves an inlet port and outlet port provided at the housing of the battery pack. These ports are shut by sealing means in regular operation of the battery pack and will only be opened for performing the inventive method for determining the composition of a gaseous mixture enclosed within a gas-tight housing. The inlet port and outlet port are placed on positions that can be opened and connected for the gas test without demounting the whole battery pack. According to a preferred embodiment, a venting opening can be used as inlet port for the inventive gas detection method. The test system creates a controlled airflow throw the battery pack, for example, by a vacuum pump or a compressor that are connected externally to the battery pack, i.e. an air stream entering the housing through the air inlet port and leaving the housing at the air outlet port will be generated. The emitted fumes and gas contents will be transported through a gas analyzer, for example a gas sensor array. By analyzing the emission, the health status is detected. For example, if H₂ is detected, the test system may announce a striking warning. The gas analyzer is directly connected to the air outlet port.

According to one embodiment, an inlet filter unit is connected to the air inlet port. Thereby, the introduction of particles or dust into the housing of the battery pack could be avoided. In other words, any unintended flow of particles or fumes from the surrounding environment inside the battery pack is prevented by an air filter which is installed in front of the inlet port.

The air stream may be produced by connecting a compressor to the air inlet port or by connecting a vacuum pump to the gas analyzer. Such an embodiment allows a simple and cost-effective implementation of the test system.

According to another preferred embodiment, an outlet filter unit for adsorbing or degrading harmful compounds is positioned downstream the gas analyzer. Thereby, emission of for example toxic gas compounds into the surrounding environment could be prevented. Thus, the outlet filter may for example include activated carbon for absorbing toxic compounds or catalyst for destructing these compounds into harmless products.

The gas analyzer may include means for qualitatively and/or quantitatively determining at least one of H₂, CO₂, CO, NOₓ, O₂, O₃, water vapor, and hydrocarbons. These compounds are indicative for the health status of the battery pack and selective gas sensors for determination are state of the art so as to cost-effective realize a suitable gas analyzer.

The gas-tight housing for a battery pack of a vehicle includes an air inlet port and an air outlet port, which are sealed by removable sealing means. In other words, the gas-tight housing is modified for performing the above method for determining the composition of a gaseous mixture enclosed within the gas-tight housing. The sealing means are screwed sealing plugs. Thereby, implementation of the air inlet port and air outlet port is very simple and opening these ports may be achieved by use of common tools, like a simple screw driver.

The air inlet port and the air outlet port may be positioned at opposite sidewalls of the housing. In addition or in alternative, the air inlet port and the air outlet port are spaced from each other such that an air stream introduced into the air inlet port runs through at least 50 percent of a total free volume of the battery pack. Thus, a path length of the air stream through the interior of the housing should be as long as possible to ensure that the health status the total battery pack could be determined.

Further aspects of the present invention could be learned from the dependent claims or the following description.

### Brief Description of the Drawings

Features will become apparent to those of ordinary skill in the art by describing in detail exemplary embodiments with reference to the attached drawings in which:
- Fig. 1: illustrates a view on a vehicle including a battery pack; and
- Fig. 2: illustrates a schematic view on a test system according to an embodiment of the present invention.

### Detailed Description of the Invention

Features of the inventive concept and method of accomplishing the same may be understood more readily by reference to the following detailed description of an embodiment and the accompanying drawings. The present invention, however, may be embodied in various different forms, and should not be construed as being limited to only the illustrated embodiment herein. Rather, this embodiment is provided as examples so that this disclosure will be thorough and complete, and will fully convey the aspects and features of the present invention to those skilled in the art.

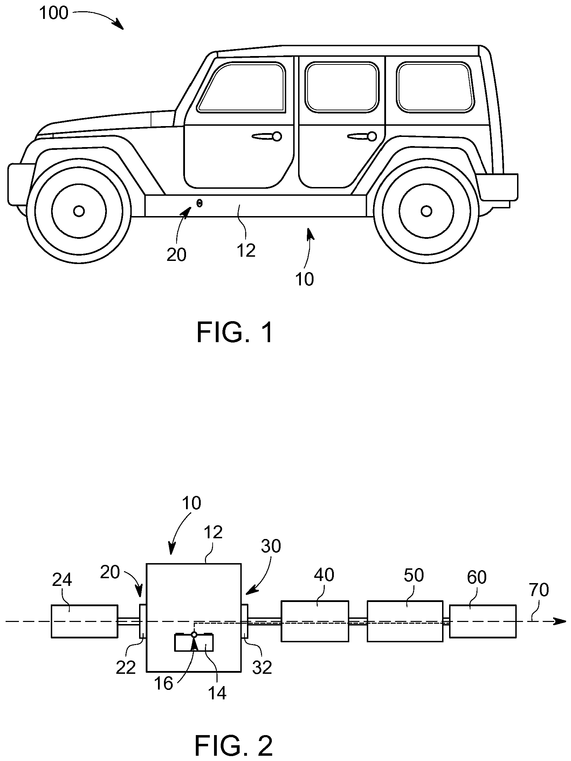

Fig. 1 illustrates an exemplary electric vehicle 100 including a battery pack 10 installed at a bottom of the vehicle's chassis. The battery pack 10 could be any of the prior art. Such battery packs 10 comprise a battery pack housing 12 that serves as enclosure to seal the battery pack 10 against the environment and provides structural protection of the battery pack's components. Housed battery packs are usually mounted as a whole into their application environment, e.g. an electric vehicle. Li-ion battery modules for EV's are placed inside the common battery pack housing. According to the exemplary embodiment, an air inlet port 20 is provided at a side wall of the battery pack housing 12. Here, the air inlet port 20 is sealed by a corresponding sealing mean, a screwed sealing plug. At an opposite side wall of the housing 12 an air outlet port is provided (not shown), which is also secured in usual operation by a screwed sealing plug.

Fig. 2 schematically illustrates an embodiment of a test system for determining a composition of a gaseous mixture enclosed within the gas-tight housing 12 of the battery pack 10. For purpose of illustration, the battery pack 10 includes a battery cell 14 with a leakage defect 16 of its casing such that an electrolyte may leak into the interior of the housing 12. The battery cell 14 may be damaged by for example a crash.

So as to get information on the operation status of the battery pack 10, a rescue team will mount the inventive test system illustrated in the exemplary embodiment of Fig. 2. At first, the screwed sealing plug at the air inlet port 20 of the housing 12 will be removed by the rescue team and an inlet connection pipe 22 will be mounted instead of. Furthermore, an inlet filter unit 24 is provided upstream the inlet connection pipe 22 such that air introduced through the air inlet port 20 is filtered and, for example, no particles are sucked into the interior of the housing 12.

At an opposite side of the housing 12 the air outlet port 30 is opened by the rescue team and an outlet connection pipe 32 is installed. Downstream thereof, a gas analyzer 40, a vacuum pump 50 and an outlet filter unit 60 are provided. The gas analyzer 40 includes for example a gas sensor array for qualitatively and/or quantitatively determining one or more of H₂, CO₂, CO, NOₓ, O₂, O₃, water vapor, and hydrocarbons.

The gas analyzer 40 will further include appropriate means for evaluation and, if necessary, visual or acoustic indications of a dangerous state of the battery pack 10. For example, a H₂-sensitive gas sensor may be arranged in the air flow and when the H₂ content exceeds a predetermined threshold, the analyzer will initiate an acoustic waring signal.

The vacuum pump 50 deals for generating an air stream 70 through the housing 12. In addition or in alternative, a compressor may be connected to the inlet connection pipe 22 at the air inlet port 20.

In the exemplary embodiment, the outlet filter unit 60 is mounted downstream the vacuum pump 50. However, in order to avoid any unwanted interaction of harmful compounds it is preferred to mount the outlet filter unit 60 between the gas analyzer 40 and the vacuum pump 50. The outlet filter unit 60 includes for example absorbing materials, like activated carbon, and/or catalysts for destructing of toxic or explosive compounds into harmless products, for example oxidation catalysts. The outlet filter unit 60 may, for example, also include a particle filter.

When the mentioned components of the test system are mounted to the battery pack 10, the vacuum pump 50 is switched on and an air stream 70 will flow through the assembly starting from the inlet filter unit 24 and then flowing subsequently through the inlet connection pipe 22, the interior of the housing 12, outlet connection pipe 32, gas analyzer 40, vacuum pump 50 and leaving the test system through the outlet filter unit 60. When passing the housing 12, the air stream 70 will carry with vaporized electrolyte of the defect battery cell 14. Common lithium secondary batteries will include for example hydrocarbons which may then be detected with the gas analyzer 40 and afterwards absorbed in the outlet filter unit 60. By positioning the air inlet port 20 and air outlet port 30 at opposite sides of the housing 12, the air stream 70 passes large areas of the interior of the housing 12.

### Reference signs

- 10: battery pack
- 12: battery module
- 14: battery cell
- 16: leakage defect of the battery cell 14
- 20: air inlet port
- 22: inlet connection pipe
- 24: inlet filter
- 30: air outlet port
- 32: outlet connection pipe
- 40: gas analyzer
- 50: vacuum pump
- 60: outlet filter
- 70: gas stream
- 100: vehicle

## Claims

1. A method for determining the composition of a gaseous mixture enclosed within a gas-tight housing (12) of a battery pack (10) for a vehicle (100), wherein the housing (12) includes an air inlet port (20) and an air outlet port (30), which are sealed by removable sealing means, wherein the sealing means are screwed sealing plugs, the method comprising the steps of:
a) removing the screwed sealing plugs from the air inlet port (20) and from the air outlet port (30);
b) generating an air stream (70) entering the housing (12) through the air inlet port (20) and leaving the housing (12) at the air outlet port (30); and
c) analyzing the air stream (70) leaving the air outlet port (30) with a gas analyzer (40).

2. The method of claim 1, wherein an inlet filter unit (24) is connected to the air inlet port (20).

3. The method of any one of the preceding claims, wherein the air stream is produced by connecting a compressor to the air inlet port (20) or by connecting a vacuum pump (50) to the gas analyzer (40).

4. The method of any one of the preceding claims, wherein a filter unit for adsorbing or degrading harmful compounds is positioned downstream the gas analyzer (40).

5. The method of any one of the preceding claims, wherein the gas analyzer includes means for qualitatively and/or quantitatively determining at least one of H₂, CO₂, CO, NOₓ, O₂, O₃, water vapor, and hydrocarbons.

## Patentansprüche

1. Verfahren zur Bestimmung der Zusammensetzung eines Gasgemisches, das in einem gasdichten Gehäuse (12) eines Batteriepacks (10) für ein Fahrzeug (100) eingeschlossen ist, wobei das Gehäuse (12) eine Lufteinlassöffnung (20) und eine Luftauslassöffnung (30) umfasst, die durch entfernbare Dichtungsmittel abgedichtet sind, wobei die Dichtungsmittel geschraubte Dichtungsstopfen sind, wobei das Verfahren die folgenden Schritte umfasst:
a) Entfernen der geschraubten Dichtungsstopfen von der Lufteinlassöffnung (20) und von der Luftauslassöffnung (30);
b) Erzeugen eines Luftstroms (70), der in das Gehäuse (12) durch die Lufteinlassöffnung (20) eintritt und das Gehäuse (12) an der Luftauslassöffnung (30) verlässt; und
c) Analysieren des Luftstroms (70), der die Luftauslassöffnung (30) verlässt, mit einem Gasanalysator (40).

2. Verfahren nach Anspruch 1, wobei eine Einlassfiltereinheit (24) mit der Lufteinlassöffnung (20) verbunden ist.

3. Verfahren nach einem der vorhergehenden Ansprüche, wobei der Luftstrom durch Anschluss eines Kompressors an die Lufteinlassöffnung (20) oder durch Anschluss einer Vakuumpumpe (50) an den Gasanalysator (40) erzeugt wird.

4. Verfahren nach einem der vorhergehenden Ansprüche, wobei dem Gasanalysator (40) eine Filtereinheit zur Adsorption oder zum Abbau von Schadstoffen nachgeschaltet ist.

5. Verfahren nach einem der vorhergehenden Ansprüche, wobei der Gasanalysator Mittel zur qualitativen und/oder quantitativen Bestimmung von mindestens einem der folgenden Elemente enthält: H₂ , CO₂ , CO, NOₓ , O₂ , O₃ , Wasserdampf und Kohlenwasserstoffe.

## Revendications

1. Procédé de détermination de la composition d'un mélange gazeux enfermé dans un logement étanche au gaz (12) d'un bloc-batterie (10) pour un véhicule (100), dans lequel le logement (12) inclut un orifice d'entrée d'air (20) et un orifice de sortie d'air (30), qui sont scellés par des moyens d'étanchéité amovibles, dans lequel les moyens d'étanchéité sont des bouchons d'étanchéité vissés, le procédé comprenant les étapes consistant à :
a) retirer les bouchons d'étanchéité vissés de l'orifice d'entrée d'air (20) et de l'orifice de sortie d'air (30) ;
b) générer un courant d'air (70) entrant dans le logement (12) à travers l'orifice d'entrée d'air (20) et quittant le logement (12) au niveau de l'orifice de sortie d'air (30) ; et
c) analyser le courant d'air (70) quittant l'orifice de sortie d'air (30) avec un analyseur de gaz (40).

2. Procédé selon la revendication 1, dans lequel une unité de filtre d'entrée (24) est reliée à l'orifice d'entrée d'air (20).

3. Procédé selon l'une quelconque des revendications précédentes, dans lequel le courant d'air est produit par liaison d'un compresseur à l'orifice d'entrée d'air (20) ou par liaison d'une pompe à vide (50) à l'analyseur de gaz (40) .

4. Procédé selon l'une quelconque des revendications précédentes, dans lequel une unité de filtre pour adsorber ou dégrader des composés nocifs est positionnée en aval de l'analyseur de gaz (40).

5. Procédé selon l'une quelconque des revendications précédentes, dans lequel l'analyseur de gaz inclut des moyens pour déterminer qualitativement et/ou quantitativement au moins un parmi H₂, CO₂, CO, NOₓ, O₂, O₃, de la vapeur d'eau et des hydrocarbures.
